# EUROPEAN PATENT SPECIFICATION

(11) **EP 3 079 629 B1**
(45) Date of publication and mention of the grant of the patent: **31.01.2018**
(21) Application number: 14821385.3
(22) Date of filing: 08.12.2014
(51) Int. Cl.: A61C 9/00, A61C 17/02

(54) **DEVICE FOR DISPENSING A DENTAL PASTE, AN AIR STREAM AND A MEDICAMENT**
VORRICHTUNG ZUR AUSGABE EINER DENTALEN PASTE, EINES LUFTSTROMS UND EIN MEDIKAMENT
DISPOSITIF PERMETTANT DE DISTRIBUER UNE PÂTE DENTAIRE, UN FLUX D'AIR ET UN MÉDICAMENT

(30) Priority: 13.12.2013 EP 13197092
(43) Date of publication of application: 19.10.2016
(73) Proprietor: 3M Innovative Properties Company, St. Paul, MN 55133-3427 (US)
(72) Inventor: MAURER, Andreas, 82229 Seefeld (DE); SCHULTE, Christoph, 82229 Seefeld (DE); PAUSER, Helmut, 82229 Seefeld (DE); HAMPE, Ruediger, 82229 Seefeld (DE); LEYKAUFF, Johannes, 82229 Seefeld (DE); PEUKER, Marc, 82229 Seefeld (DE)
(74) Representative: Hohmann, Arno
(86) International application number: PCT/US2014/069053
(87) International publication number: WO 2015/088959

(56) References cited:
- EP-A1- 0 296 347
- EP-A2- 1 468 659
- WO-A2-2012/114337
- US-A- 3 530 587
- US-A- 3 854 209
- US-A- 4 264 305
- US-A- 5 409 465
- US-A- 5 820 373

## Description

### Field of the Invention

The invention relates to device for dispensing a dental paste, and in particular to a device having a first nozzle and a second nozzle for simultaneously delivering a dental paste and a gas or air stream toward a working area. Further the invention relates to a method of performing a dental treatment by simultaneously delivering a dental paste and a gas stream and in a patient's mouth.

### Background Art

The restoration of a patient's tooth or teeth often includes the replacement of natural tooth structure by a manufactured dental restoration or dental prosthesis. Typically a tooth that is to be restored is first prepared by a dentist, for example by grinding to remove undesired tooth substance, and to provide the tooth with a shape appropriate to receive the dental restoration.

The dental restoration is typically designed to precisely fit onto the residual tooth substance on the basis of a dental impression previously taken from the prepared tooth. The dental impression should be very precise, and should represent all tooth structure required to determine the shape of the later dental restoration. In particular the dental impression should represent the transition or the "margin" between the residual tooth structure and the dental restoration. For dental restorations that extend below a patient's gingiva (or gums) the dentist also should make the part of the margin accessible for the dental impression that would normally be covered by the gingiva. The procedure of displacing the gingiva from the tooth to make the margin accessible is also known as "gingival retraction" in the field of dentistry. A common gingival retraction procedure includes the insertion of a so called retraction cord in the gingival sulcus.

A further gingival retraction procedure is based on the use of so-called gingival retraction pastes. Typically retraction pastes are very high viscosity pastes which - instead of a cord - are typically squeezed in the gingival sulcus in a patient's mouth. In one example a paste is provided which is supposed to be brought into a patient's gingival sulcus to displace the gingival tissue from a tooth. A known paste is for example available under the designation Expasyl, from the company Pierre Roland, Acteon Group, France.

Still further WO 2009/151983 A2 discloses device for use in retracting a gingiva by widening a gingival sulcus with a dental composition. The device comprises a cannula with a free end having an opening for dispensing the dental composition. The free end is shaped to be inserted with its front in the entry of the gingival sulcus, and to laterally displace the gingiva from the tooth as the cannula is moved in the gingival sulcus. US3854209 A1, considered as being the closest prior art document, discloses a device for dispensing a dental retraction material, comprising a first nozzle for delivering the dental retraction material and a second and third nozzles for delivering a gas stream, wherein the device is adapted for simultaneously delivering the gas stream and the dental retraction material toward a working area, wherein the device comprises a reservoir for the dental retraction material, and at least one piston for extruding the dental retraction material, wherein the dental retraction material and the gas stream each are individually supplied toward the first nozzle and the second and third nozzles, respectively, wherein the first nozzle and the second and third nozzles are oriented such that the delivered dental retraction material and the gas stream join each other outside the device, wherein the first nozzle has a first outlet and the second and third nozzles has a second and third outlets, and wherein the first and second and third outlets face toward a common point or area.

Although existing gingival retraction procedures and useful devices therefor provide certain advantages there is still a need for a procedure and a device allowing for convenient insertion of a composition for gingival retraction into the gingival sulcus. It is particularly desirable that a gingival retraction procedure can be performed in an acceptably short time, and with relatively little effort by the dentist. Further there is a need for a procedure that provides a treatment that is reliable, acceptable for a patient, and has few adverse side-effects or after-effects. It is further desirable to provide a gingival retraction device and composition that are rather inexpensive.

### Summary of the Invention

In one aspect the invention relates to a device for dispensing a dental paste. The device comprises a first nozzle for delivering the paste and a second nozzle for delivering a gas or air stream. The device is adapted for simultaneously delivering the gas/air stream and the paste toward a working area.

The invention may be advantageous for taking a dental impression, in particular a dental impression extending beneath the gingival margin in a patient's mouth. The invention helps removing undesired substances, like saliva and/or blood, from a working area in a patient's mouth by way of the gas or air stream short before delivering the dental paste, for example a dental impression material, to the same working area. In particular the invention preferably allows delivering the dental paste and the gas/air stream by the same device and within one single step of treatment. Therefore the invention further helps minimizing costs during a dental treatment and additionally helps maximizing the quality of the dental treatment.

In the embodiment of the invention, the paste and the gas/air stream each are individually supplied toward the first and second nozzle, respectively. In particular the paste and the gas/air may meet only outside the device. Thus neither of the paste of the gas/air stream may become contaminated before delivery out of the device. The first nozzle and the second nozzle are oriented such that the delivered paste and the gas stream join each other outside the device. In particular the first nozzle has a first outlet and the second nozzle has a second outlet, and the first and second outlet face toward a common point or the working area. The working area may have a dimension within a range of 1 mm² to 25 mm², and in more particular within a range of 1 mm² to 10 mm². This helps for example displacing any undesired liquids from a working area sufficiently short before delivering the dental paste to the same working area while the device is moved, for example by a dental practitioner.

In one embodiment of the invention, the first outlet and the second outlet are arranged side by side.
Preferably the second outlet is arranged offset from the first outlet in a direction upstream of the gas stream. Thus the device is adapted for inserting the first nozzle in a cavity such that the second nozzle is located outside that cavity. This helps avoiding any paste delivered to the cavity to be displaced by the gas/air stream.

In an alternative embodiment of the invention, the first outlet and the second outlet are arranged coaxially relative to each other. In this embodiment the second outlet surrounds the first outlet. Further the second outlet is preferably arranged offset from the first outlet in a direction upstream of the gas stream.

In the embodiment of the invention, the device comprises a third nozzle for delivering a liquid or a powdery medicament. The third nozzle may open into a gas supply for the gas stream. Further third nozzle may open into the second nozzle. The second nozzle may form a spray nozzle allowing the medicament to be dispersed in the gas/air stream. The device may have a container for receiving the medicament and a medicament supply connecting the container and the third nozzle. The medicament may comprise hemostasis and/or anesthesia.

In the embodiment of the invention, the device comprises a reservoir for the paste or a reservoir for components from which the paste can be mixed. The device further has at least one piston for extruding the paste or a component thereof from the reservoir. The device may comprise a container instead of a reservoir, wherein the container may be replaceably receivable within the device, whereas the reservoir may be integral with the device and may be refillable.

In one embodiment the device, in particular the reservoir or the container, comprises a dental impression material or a dental retraction material.
In a further aspect the disclosure relates to a method of performing a dental treatment.
The method comprises the steps of:
- delivering a gas stream toward a working area in a patient's mouth; and
- delivering a dental paste to the same working area;
- wherein the delivery of the gas stream and the paste are performed at least over a partial time period of the delivery simultaneously.

In one embodiment the working area is a sulcus of the patient or a cavity in the patient's mouth. The method may further comprise the step of delivering a medicament, for example hemostasis or anesthesia toward the working area.

### Brief Description of the Figures

- Fig. 1: illustrates a stage of a method of use of the device according to an embodiment of the invention;
- Fig. 2: is a cross-sectional view of a device according to an embodiment of the invention;
- Fig. 3: is a cross-sectional partial view of a device according to a further embodiment of the invention; and
- Fig. 4: is a cross-sectional partial view of a device according to still a further embodiment of the invention.

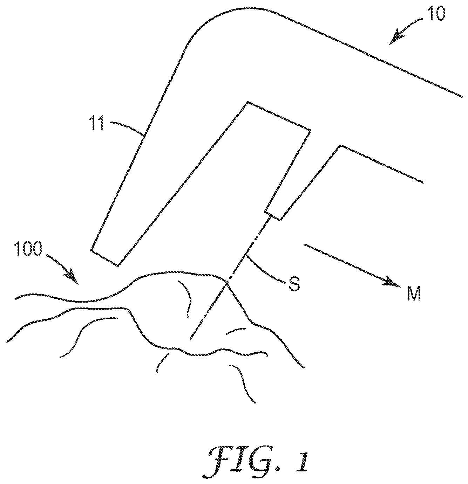

Figures 1 to 4 do not disclose the claimed subject-matter.

### Detailed Description of the Invention

Fig. 1 illustrates a treatment of a patient's sulcus 100 for dental retraction. In particular the dental retraction in the example is based on dispensing a paste (at a stage not shown) into the patient's sulcus. A device 10 of the invention is used to open the sulcus 200 by a gas or air stream S which is directed toward the opening of the sulcus 200. The velocity of the gas/air stream is adjusted such that the opening of the sulcus 200 widens from the air/gas stream.

Fig. 1 illustrates a situation in which the sulcus 200 is sufficiently widened by the gas/air stream such that a nozzle 11 of the device can be easily inserted into the sulcus 100 for delivering paste in to the sulcus 100. Further it has been found that the gas/air stream is advantageous in that it helps displacing liquids (for example saliva and/or blood) from the sulcus and thus helps cleaning the sulcus for receiving the paste therein. This is of particular advantage in case a dental impression material is delivered in the sulcus because this allows taking a relatively precise dental impression of a tooth including areas beneath the patent's gingival margin (with minimized liquid hindering the impression material to replicate any structures). Accordingly during the treatment the gas/air stream is preferably delivered simultaneously with delivering paste. In the example the device 10 may be moved in a direction o "M" so that paste is delivered to areas which previously have been exposed to the air stream. Therefore the paste is preferably received in areas from which any liquids have already been displaced by the gas/air stream.

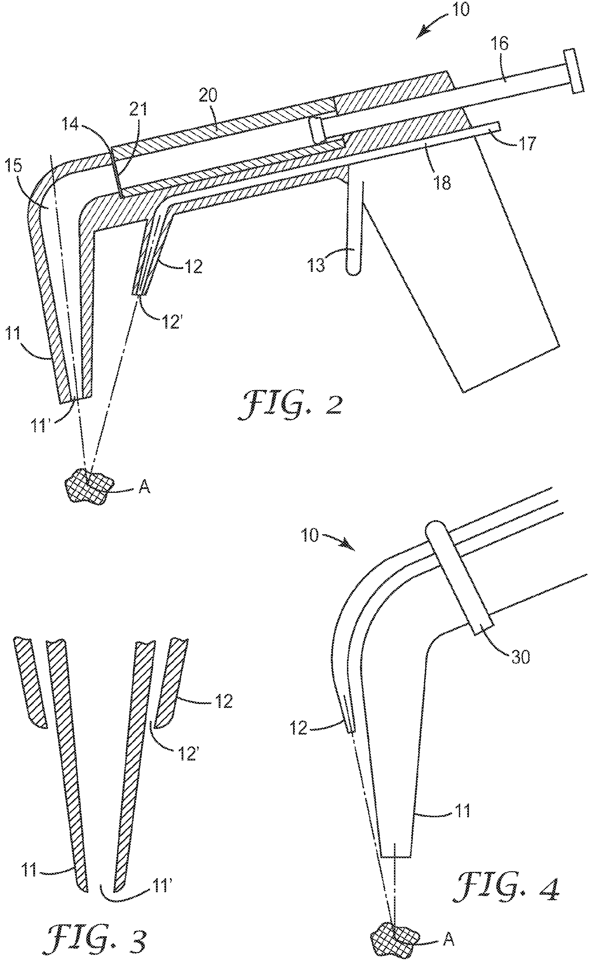

Fig. 2 shows device 10 for dispensing a dental paste. The device 10 comprises a first nozzle 11 for delivering the paste and a second nozzle 12 for delivering a gas stream. In the example the device 10 is adapted for delivering air through the second nozzle 12. The device 10 is adapted for simultaneously delivering the gas or air stream and the paste toward a working area A.

The device 10 of the example is a hand-held device which has an actuator 13 for activating the dispensation of paste and the delivery of the air stream. In more particular the device 10 of the example is adapted such the actuator 13 is operable (for example by a user of the device 10) between an off position, in which any delivery of paste and air is deactivated, an intermediate position in which only the delivery of the air stream is activated, and a dispensing position in which paste and the air stream are delivered. In another example the device 10 may be adapted such the actuator 13 is operable only between the off position and the dispensing position, and an intermediate position may not be available.

In the device 10 the paste and the gas stream each are individually supplied toward the first nozzle 11 and second nozzle 12, respectively. In particular the device 10 has a receptacle 14 for receiving a container 20 containing the paste. The container may be received within the device 10 such that an exit 21 of the container 20 is in fluid communication with a passageway 15 of the device 10. The passageway 15 forms a first outlet 11' in the first nozzle 11 for the paste. The passageway 15 is thus adapted to guide paste extruded from the container 20 to the first outlet 11' of the first nozzle 11. The device 10 of the example has a plunger 16 which is movable into the container 20 from a side opposite of the exit 21 of the container 20 for extruding the paste therefrom. Although not illustrated in detail, the device 10 may be adapted for driving the plunger 16 by motor or spring force, by gas or air pressure, or by a mechanism operated via actuation of the actuator 13. The container 20 is preferably replaceably received within the device 10. Thus an empty container may be easily replaced by a new container filled with paste.

The device 10 further has a supply connector 17 for connecting the device 10 with an air or gas supply. The supply connector 17 is in fluid connection with a channel 18, and the channel 18 forms a second outlet 12' in the second nozzle 12 for the air or gas.

The first nozzle 11 and the second nozzle 12 are oriented such that the delivered paste and the gas stream join each other outside the device. In the example the first and second nozzle 11, 12 are oriented such that the paste and the gas are delivered toward the same working area A. The first nozzle 11 and the second nozzle 12, and also the first and second outlet 11', 12', face toward a common point or area. Thus the device is adapted such that a strand of paste delivered from the device 10 and the gas stream intersect outside the device.

In the example illustrated in Fig. 2 the first outlet 11 and the second outlet 12 are arranged side by side. However the second outlet 12' of the second nozzle 12 is arranged offset relative to the first outlet 11' of the first nozzle 11 in a direction upstream of the gas/air stream. Therefore the device 10 may be particularly useful for delivering paste via the first nozzle 11 into a cavity, for example a tooth cavity of a gingival sulcus. This is because the first nozzle 11 may be shaped such that it can be inserted in narrow locations while the second nozzle 12 can be kept outside such location.

Although not illustrated the device 10 is adapted for delivering a liquid or a powdery medicament through the second nozzle 12. A third nozzle (not shown) is arranged within the second nozzle 12 and the delivery of gas/air may cause (for example by means of a Venturi arrangement of the nozzles) the medicament to disperse within the gas/air stream. Such a medicament may comprise hemostasis and/or anesthesia, for example.

Fig. 3 shows an alternative configuration of the first nozzle 11 and the second nozzle 12. In this example the first nozzle 11 and the second nozzle 12 are arranged coaxially relative to each other. Again the second outlet 12' of the second nozzle 12 is arranged offset relative to the first outlet 11' of the first nozzle 11 in a direction upstream of the gas/air stream.

Fig. 4 shows a portion of a device 10 in which the second nozzle 12 is removably attached to the device 10. In this example the first and second nozzle 11, 12 are arranged side by side and oriented toward the same point or area A in a situation in which the second nozzle 12 is appropriately attached to the device 10. For attachment of the second nozzle 12 a clamp 30 is provided which in the example is fixed to the first nozzle 11 and holds the second nozzle 11 in place.

The second nozzle in any of the embodiments disclosed herein preferably has an open cross-section of between about 0.03 mm² and 78.5 mm².

## Claims

1. A device (10) for dispensing a dental retraction material, comprising a first nozzle (11) for delivering the dental retraction material and a second nozzle (12) for delivering a gas stream, wherein the device (10) is adapted for simultaneously delivering the gas stream and the dental retraction material toward a working area, wherein the device (10) comprises a reservoir (20) for the dental retraction material or a reservoir for components from which the dental retraction material can be mixed, and at least one piston (16) for extruding the dental retraction material or a component thereof from the reservoir (20), wherein the dental retraction material and the gas stream each are individually supplied toward the first nozzle (11) and the second nozzle (12) respectively, wherein the first nozzle (11) and the second nozzle (12) are oriented such that the delivered dental retraction material and the gas stream join each other outside the device, wherein the first nozzle (11) has a first outlet (11') and the second nozzle (12) has a second outlet (12'), and wherein the first and second outlets (11', 12') face toward a common point or area, comprising a third nozzle for delivering a liquid or a powdery medicament, and wherein the third nozzle opens into a gas supply for the gas stream or into the second nozzle.

2. The device of claim 1, wherein the first outlet and the second outlet are arranged side by side, with the second outlet being arranged offset from the first outlet in a direction upstream of the gas stream.

3. The device of claim 1, wherein the first outlet and the second outlet are arranged coaxially relative to each other, with the second outlet surrounding the first outlet and with the second outlet being arranged offset from the first outlet in a direction upstream of the gas stream.

4. The device of any of the preceding claims, having a container for receiving the medicament and a medicament supply connecting the container and the third nozzle.

5. The device of any of the preceding claims, wherein the medicament comprises hemostasis and/or anesthesia.

## Patentansprüche

1. Vorrichtung (10) zum Abgeben eines Dentralretraktionsmaterials, umfassend eine erste Düse (11) zum Zuführen des Dentalretraktionsmaterials und eine zweite Düse (12) zum Zuführen eines Gasstroms, wobei die Vorrichtung (10) dazu angepasst ist, gleichzeitig den Gasstrom und das Dentalretraktionsmaterial einem Arbeitsbereich zuzuführen, wobei die Vorrichtung (10) einen Behälter (20) für das Dentalretraktionsmaterial oder einen Behälter für Komponenten, aus denen das Dentalretraktionsmaterial gemischt werden kann, und mindestens einen Kolben (16) zum Extrudieren des Dentalretraktionsmaterials oder einer Komponente davon aus dem Behälter (20) umfasst, wobei das Dentalretraktionsmaterial und der Gasstrom jeweils einzeln der ersten Düse (11) bzw. der zweiten Düse (12) zugeführt werden, wobei die erste Düse (11) und die zweite Düse (12) so ausgerichtet sind, dass das zugeführte Dentalretraktionsmaterial und der Gasstrom sich außerhalb der Vorrichtung verbinden, wobei die erste Düse (11) einen ersten Auslass (11') aufweist und die zweite Düse (12) einen zweiten Auslass (12') aufweist, und wobei der erste und der zweite Auslass (11', 12') in Richtung eines gemeinsamen Punkts oder Bereichs weisen, umfassend einen dritten Auslass zum Zuführen einer Flüssigkeit oder eines pulverförmigen Medikaments, und wobei die dritte Düse in eine Gaszuleitung für den Gasstrom oder in die zweite Düse mündet.

2. Vorrichtung nach Anspruch 1, wobei der erste Auslass und der zweite Auslass nebeneinander angeordnet sind, wobei der zweite Auslass in einer dem Gasstrom vorgelagerten Richtung zum ersten Auslass versetzt angeordnet ist.

3. Vorrichtung nach Anspruch 1, wobei der erste Auslass und der zweite Auslass koaxial zueinander angeordnet sind, wobei der zweite Auslass den ersten Auslass umgibt und wobei der zweite Auslass in einer dem Gasstrom vorgelagerten Richtung zum ersten Auslass versetzt angeordnet ist.

4. Vorrichtung nach einem der vorstehenden Ansprüche, die einen Behälter zur Aufnahme des Medikaments und eine Medikamentenzuleitung aufweist, die den Behälter und die dritte Düse verbindet.

5. Vorrichtung nach einem der vorstehenden Ansprüche, wobei das Medikament Hämostase und/oder Anästhesie umfasst.

## Revendications

1. Dispositif (10) pour distribuer un matériau de rétraction dentaire, comprenant une première buse (11) pour acheminer le matériau de rétraction dentaire et une deuxième buse (12) pour acheminer un courant gazeux, où le dispositif (10) est conçu pour acheminer simultanément le courant gazeux et le matériau de rétraction dentaire vers une zone de travail, où le dispositif (10) comprend un réservoir (20) pour le matériau de rétraction dentaire ou un réservoir pour des composants à partir desquels le matériau de rétraction dentaire peut être mélangé, et au moins un piston (16) pour extruder le matériau de rétraction dentaire ou un composant de celui-ci à partir du réservoir (20), dans lequel le matériau de rétraction dentaire et le courant gazeux sont chacun fournis individuellement vers la première buse (11) et la deuxième buse (12) respectivement, dans lequel la première buse (11) et la deuxième buse (12) sont orientées de telle sorte que le matériau de rétraction dentaire acheminé et le courant gazeux se rejoignent l'un l'autre à l'extérieur du dispositif, dans lequel la première buse (11) a une première sortie (11') et la deuxième buse (12) a une deuxième sortie (12'), et dans lequel les première et deuxième sorties (11', 12') font face à un point ou zone commun, comprenant une troisième buse pour acheminer un liquide ou un médicament pulvérulent, et dans lequel la troisième buse s'ouvre dans une alimentation de gaz pour le courant gazeux ou dans la deuxième buse.

2. Dispositif selon la revendication 1, dans lequel la première sortie et la deuxième sortie sont disposées côte à côte, la deuxième sortie étant disposée décalée de la première sortie dans une direction en amont du courant gazeux.

3. Dispositif selon la revendication 1, dans lequel la première sortie et la deuxième sortie sont disposées de manière coaxiale l'une par rapport à l'autre, la deuxième sortie entourant la première sortie et la deuxième sortie étant disposée décalée de la première sortie dans une direction en amont du courant gazeux.

4. Dispositif selon l'une quelconque des revendications précédentes, possédant un récipient pour recevoir le médicament et une alimentation en médicament reliant le récipient et la troisième buse.

5. Dispositif selon l'une quelconque des revendications précédentes, dans lequel le médicament comprend une hémostase et/ou une anesthésie.
